(19) Europäisches Patentamt
European Patent Office
Office européen des brevets

(11) EP 0 862 909 A2

## (12) EUROPEAN PATENT APPLICATION

(43) Date of publication:
09.09.1998 Bulletin 1998/37

(21) Application number: 98200795.7

(22) Date of filing: 25.02.1993

(51) Int Cl.$^6$: **A61K 7/16**, A61K 7/22

(84) Designated Contracting States:
AT BE CH DE DK ES FR GB GR IE IT LI LU MC NL PT SE

(30) Priority: 29.02.1992 GB 9204410

(62) Document number(s) of the earlier application(s) in accordance with Art. 76 EPC:
93904254.5 / 0 627 908

(71) Applicant: SMITHKLINE BEECHAM PLC
Brentford, Middlesex TW8 9EP (GB)

(72) Inventors:
• Barnett, Paul, c/o Smithkline Beecham Pharma.
Weybridge, Surrey, KT13 0DE (GB)

• Burgon-Lyon, Kristy
Weybridge, Surrey, KT13 0DE (GB)

(74) Representative: Thompson, Clive Beresford et al
SmithKline Beecham plc
Corporate Intellectual Property,
Two New Horizons Court
Brentford, Middlesex TW8 9EP (GB)

Remarks:
This application was filed on 12 - 03 - 1998 as a divisional application to the application mentioned under INID code 62.

## (54) Compositions

(57) Oral hygiene compositions are described which comprise a cationic anti-bacterial agent and polyvinyl pyrrolidone, to counter the staining associated with use of a a cationic anti-bacterial agent.

## Description

The present invention relates to oral hygiene compositions comprising a cationic anti-bacterial agent and an anti-staining agent, for use in treating or preventing dental plaque, caries, or periodontal diseases of the oral cavity in humans or lower animals with reduced staining of teeth.

Cationic antibacterial agents such as chlorhexidine have long been recognised as useful therapeutic agents for treating plaque, caries and peridontal diseases due not only to their intrinsic *in vitro* antibacterial activity against strains implicated in the formation of plaque but also because of their substantivity to oral tissue. They are thus adsorbed onto oral surfaces and gradually released over a period of time, to produce a prolonged antibacterial effect and hence reduce or prevent the formation of plaque over a period of time. It is believed that the deposition of plaque onto the tooth surface is one of the first steps in the development of dental caries and periodontal disease.

It is however also widely recognised that the use of cationic antibacterial agents in oral hygiene, in particular chlorhexidine, is accompanied by the formation of a cosmetically disfiguring brown stain on the tooth surfaces. This is believed to arise from an interaction between the cationic antibacterial agent and certain dietary components, particularly those rich in tannins such as tea, coffee and red wine. Whilst this problem may to some extent be alleviated by brushing of the teeth with a toothpaste prior to use of the product containing the cationic antibacterial agent, in many instances it is found necessary to resort to professionally administered scaling and polishing to remove the stain.

In consequence there have been many suggestions in the patent literature for chemical antistaining agents which maybe incorporated with the cationic antibacterial agent or which may, in some instances, be used instead separately, after the cationic antibacterial agent. Examples include carboxylic acids (US 4 256 731), aminocarboxylate compounds (US 4 080 441), phosphonoacetic acid (US 4 118 474), peroxydiphosphate salts (US 4 273 759) and, more recently, a polymer having certain pendant polyalkylene oxide chains (GB 2 213 721-A).

We have now surprisingly found that the development of stain may be at least substantially mitigated if not eliminated by the use of another polymeric material *viz* polyvinylpyrrolidone (PVP). This material is already used in some toothpastes at comparatively low levels (typically about 0.1%) as an auxiliary thickening agent and foam enhancer. In addition, it has been previously reported that PVP may be used in oral hygiene compositions, for the prevention of stains associated with the use of chlorophyll in such compositions (GB 739 936, Colgate-Palmolive Co) and also in the removal of stains, in particular tobacco tar stains, from tooth surfaces (GB 741 315, Colgate-Palmolive-Peet Co).

Accordingly, the present invention provides an oral hygiene composition comprising a bacteriostatic effective amount of a cationic anti-bacterial agent, an anti-stain effective amount of an anti-staining agent and an orally acceptable carrier or excipient, characterised in that the anti-staining agent is polyvinyl pyrrolidone.

Polyvinyl pyrrolidone for use in the present invention suitably has an average molecular weight in the range 5,000 to 100,000, preferably in the range 5,000 to 50,000. Polyvinyl pyrrolidones which have average molecular weights of 10,000, 30,000 and 40,000 are available from Sigma Chemical Co., GAF Corporation and Sigma Chemical Co. respectively. Polyvinyl pyrrolidone is suitably present in at least 1%, preferably between 2 and 30%, more preferably between 5 and 25% , and advantageously between 10 and 25% by weight of the composition.

Suitable cationic antibacterial agents for use in the present invention and well known in the art and include polybiguanides, in particular *bis*-biguanides such as chlorhexidine and alexidine; quaternary ammonium compounds such as cetyltrimethylammonium salts, benzalkonium salts and benzethonium salts; pyridinium compounds such as cetyl pyridinium salts, including cetyl pyridinium chloride; *bis*-pyridinamine derivatives such as octenidine; pyrimidine derivatives such as hexitidine; amidine derivatives such as hexamidine; and where appropriate and not already so indicated, orally acceptable acid addition salts thereof. Suitably, the cationic anti-bacterial agent is present in from 0.005 to 10% prefeably 0.01 to 5%, more preferably 0.01 to 2%, by weight of the oral hygiene composition.

Preferably, the cationic antibacterial agent is an orally acceptable cetyl pyridinium salt such as cetyl pyridinium chloride, or chlorhexidine or an orally acceptable acid addition salt thereof. Suitable such salts of chlorhexidine include those which have a solubility at 20°C of at least 0.005% w/v and include the digluconate, diformate, diacetate, dipropionate, dihydroiodide, dihydrochloride, dilactate, dinitrate, sulphate, and tartrate. Of these, the digluconate, diacetate and dihydrochloride are favoured.

Oral hygiene compositions of the present invention may also usefully contain an ionic fluorine-containing compound. Suitable ionic fluorine-containing compounds include, for instance, fluoride salts such as amine fluorides and alkali metal fluoride salts, for example sodium fluoride, and monofluorophosphate salts such as alkali metal monofluorophosphate salts, for example sodium monofluorophosphate. Suitably the ionic fluorine-containing compound is incorporated into the composition to provide between 100 and 3000ppm, preferably between 500 and 2000ppm of fluoride ions.

Oral hygiene compositions of the present invention maybe provided in any of the presentations normally used for such products, for instance, dentifrices including toothpastes and toothpowders, abrasive and non-abrasive gels, mouthwashes, gargles, irrigating solutions, mouthsprays and presentations for sucking or chewing by the user such as gums, pastilles and lozenges. Components for the orally acceptable carrier or excipient will be selected according to the particular type of presentation involved.

It will be appreciated by those skilled in the art that in order to ensure that the antibacterial efficacy of the cationic antibacterial agent is not substantially diminished, compatible components will be selected for inclusion in the orally acceptable carrier or excipient. Accordingly, the skilled man will readily appreciate that where necessary anionic species should be preferably avoided as such species may cause inactivation of the cationic antibacterial agent by the formation of an insoluble precipitate therewith. Thus, anionic surfactants and anionic thickening agents should preferably be rejected in favour of non-anionic counterparts such as nonionic, cationic or amphoteric surfactants and nonionic thickening agents. Similarly, abrasives capable of acting as a source of anions, either because they are sparingly soluble or whilst being essentially insoluble *per se* are contaminated with soluble impurities arising from, for instance, the manufacture thereof, are preferably avoided in favour of compatible abrasives.

Suitable compositions comprising a cationic antibacterial agent are described in EP 0 364 245-A2, EP 0 368 130-A2 and EP 0 422 803-A2.

Suitable nonionic surfactants include, for example, polyethoxylated sorbitol esters, in particular polyethoxylated sorbitol monoesters, for instance, PEG(40) sorbitan di-isostearate, and the products marketed under the trade name 'Tween' by ICI; polycondensates of ethylene oxide and propylene oxide (poloxamers), for instance the products marketed under the trade name 'Pluronic' by BASF-Wyandotte; condensates of propylene glycol; polyethoxylated hydrogenated castor oil, for instance, cremophors; and sorbitan fatty esters.

Suitable amphoteric surfactants include, for example, long chain imidazoline derivatives such as the product marketed under the trade name 'Miranol C2M' by Miranol; long chain alkyl betaines, such as the product marketed under the tradename 'Empigen BB' by Albright + Wilson, and long chain alkyl amidoalkyl betaines, such as cocamidopropylbetaine, and mixtures thereof.

Suitable cationic surfactants include the D,L-2-pyrrolidone-5-carboxylic acid salt of ethyl-N-cocoyl-L-arginate, marketed under the trade name CAE by Ajinomoto Co. Inc.

Advantageously, the surfactant is present in the range 0.005 to 20%, preferably 0.1 to 10%, more preferably 0.1 to 5% by weight of the dentifrice.

Suitable nonionic thickening agents include, for example, $(C_{1-6})$-alkylcellulose ethers, for instance methylcellulose, hydroxy$(C_{1-6})$-alkylcellulose ethers, for instance hydroxypropylcellulose, $(C_{2-6})$-alkylene oxide modified $(C_{1-6})$-alkylcellulose ethers, for instance hydroxypropyl methylcellulose, and mixtures thereof. Advantageously the nonionic thickening agent is present in the range 0.01 to 30%, preferably 0.1 to 15%, more preferbly 1 to 5%, by weight of the composition.

Suitable sparingly soluble salts that may be used as an abrasive include calcium carbonate, calcium phosphates, magnesium carbonate, insoluble sodium metaphosphate, and suitable mixtures thereof. The agent to suppress anion formation typically comprises a water soluble salt containing a cation which may be same as the cation of the abrasive and which forms an essentially insoluble or sparingly soluble salt with the anion of the abrasive. Preferably the sparingly soluble salt used as an abrasive is calcium carbonate, advantageously used in combination with dicalcium phosphate, which also usefully buffers the pH of the formulation. Suitable types of calcium carbonate include both natural and synthetic chalks. The agent to suppress anion formation may be an alkaline earth metal salt, for instance calcium chloride. The agent is preferably present in from 0.0001 to 1%, more preferably 0.005 to 0.1% by weight of the dentifrice.

The term 'essentially insoluble compound' as used herein refers to a compound which is intrinsically insoluble in aqueous solution and includes those compounds which are listed as being 'insoluble' in cold water in the 'Handbook of Chemistry and Physics', 48th Edition, Chemical Rubber Company, Section B, Physical Constants of Inorganic Compounds. Furthermore, such compounds when used as an abrasive shall contain little if any contaminating anionic impurities. Preferably the insoluble abrasive compound should contains less than 1%, preferably less than 0.5%, and more preferably less than 0.25% of anionic impurities, based on the weight of the abrasive. Suitable essentially insoluble compounds for use as abrasives include, for example, silica, zinc orthophosphate, plastics particles, alumina, hydrated alumina, and calcium pyrophosphate or mixtures thereof.

Preferably, the abrasive is silica. Suitable silicas include natural amorphous silica, for instance diatomaceous earth; and synthetic amorphous silicas, for instance a precipitated silica, or a silica gel, such as a silica xerogel; or mixtures thereof. The preferred synthetic amorphous silicas are those with a low-level of water soluble anionic impurities (hereinafter referred to as "low-anion silica"). These are obtainable from manufacturing process which are carefully controlled so that the level of anion impurities, particularly sulphate and silicate from sodium sulphate and sodium silicate, respectively, is kept to a minimum. Alternatively, or in addition, the level of anion impurities may be reduced to the required level by careful washing of the initially produced silica with, for instance, deionised or distilled water. Suitable low-anion silicas contain less than 0.5%, preferably less than 0.25%, more preferably less than 0.1% by weight of water soluble impurities such as sodium sulphate and/or sodium silicate. Examples of such low-anion silicas are described in EP A 0 315 503 (to Rhone-Poulenc). Suitable silica xerogels are described in US 3 538 230. Preferred precipitated silicas include the grade RP93 available from Rhone-Poulenc and those marketed under the trade name 'SIDENT' by Degussa, for instance, SIDENT 9 silica. Preferred silica xerogels are those marketed under the trade name 'SYLOBLANC' by W.R. Grace Corporation, Davison Chemical Division. Suitable grades of precipitated silica have BET surface areas in the range 20 to 300, preferably 20 to 100 $m^2/g$ and median agglomerate sizes in the range 2 to 50, preferably 5 to 30 $\mu$. Suitable forms of diatomaceous earth include those marketed under the trade name 'Celite' by Johns-Manville Products Corporation, for instance 'Celite Superfine Superfloss'.

The abrasive is advantageously present in the range 1 to 80%, preferably 5 to 70%, more preferably 5 to 60% by weight of the dentifrice.

It will be appreciated that each of the thickening agent, the surfactant and abrasive should be, at the level employed in the dentifrice, compatible with the cationic antibacterial agent, that is, each will not substantially reduce the availability of the cationic antibacterial agent, for instance, by more than 30%, preferably more than 20%. This may be confirmed by, for instance, determining the biological activity of the formulation, by conventional microbiological assay using, for instance, *M. luteus* as the assay organism in a standard agar diffusion method, in the presence and absence of each of the aforementioned thickening agent, surfactant and abrasive.

Mouthwashes according to the present invention will preferably comprise as components of the carrier a surfactant and a humectant in an aqueous or an aqueous/ethanol solution. Gels according to the present invention will preferably comprise as components of the carrier a surfactant, humectant, thickening agent and optionally water. Dentifrices according to the present invention will preferably comprise as components of the carrier a surfactant, humectant, thickening agent, abrasive and if necessary, water.

Suitable humectants for use in compositions of the invention include for instance glycerine, sorbitol, propylene glycol or polyethylene glycol, or mixtures thereof. The humectant may be present in the range from 5 to 70%, preferably 5 to 30%, more preferably 10 to 30% by weight of the dentifrice.

Other materials may be added to the compositions if required, for instance sweetening agents, flavouring agents, colouring and whitening agents, preservatives and emulsifiers.

The pH of a composition according to the invention will be orally acceptable and typically in the range pH 5 to 9.

Oral hygiene compositions according to the present invention may be prepared by mixing the ingredients thereof in the required proportions and in any order that is convenient and thereafter and if necessary adjusting the pH to the required value.

Oral hygiene compositions according to the present invention are of use in reducing or eliminating the stain normally asociated with the use of a cationic anti-bacterial agent. Accordingly, in a further aspect, the present invention provides for an oral hygiene composition as hereinbefore defined for use in therapy, in particular anti-plaque, anti-caries, anti-calculus and/or periodontal (including anti-gingivitis) therapy. The present invention also provides for the use of polyvinyl pyrrolidone and a cationic anti-bacterial agent in the manufacture of an oral hygiene composition for use in oral hygiene.

In oral hygiene compositions according to the present invention, the cationic antibacterial agent and the polyvinyl pyrrolidone anti-stain agent will normally be incorporated together in a single oral hygiene composition. The cationic antibacterial agent and the polyvinyl pyrrolidone anti-stain agent may however be also provided in separate oral hygiene compositions which may be used separately, simultaneously or sequentially. Accordingly, in a further aspect, the present invention provides an oral hygiene kit comprising as a first item an oral hygiene composition comprising an anti-stain effective amount of polyvinyl pyrrolidone and an orally acceptable excipient or carrier and, as a separate second item, an oral hygiene composition comprising a bacteriostatic effective amount of a cationic antibacterial agent and an orally acceptable excipient or carrier. It will be appreciated that in such an oral hygiene kit, the two oral hygiene compositions may be of the same or different character. Thus, for instance, the cationic antibacterial agent may be provided in a dentifrice whilst polyvinylpyrrolidone is provided in a mouthwash or gargle or *vice versa*. Alternatively both may be provided as, for example, a dentifrice, mouthwash or gargle.

In a further aspect, the present application further provides an oral hygiene composition comprising an antistain effective amount of polyvinyl pyrrolidone and an orally acceptable excipient or carrier.

The invention will now be illustrated by reference to the following examples.

**Example 1 - Efficacy of PVP in a stain model -** The efficacy of polyvinylpyrrolidone (PVP) in preventing the formation of stain associated with the use of chlorhexidine was evaluated *in vitro* in a model system which consisted of exposing a bovine incisor to a tea solution and then treating this with chlorhexidine in the absence or presence of polyvinylpyrrolidone.

A bovine incisor was mounted in an acrylic block and cleaned on the exposed tooth surface. The colour of this incisor was measured using a chromameter (Minolta CR200) to establish a baseline figure. The incisor was then rinsed with distilled water for 1 min and incubated in pooled filtered human saliva for 2 hour at 37°C, to induce pellicle formation. After further rinsing for 1 min with distilled water, the incisor was immersed for 2 min in a solution containing chlorhexidine digluconate (1% in 0.1M sodium acetate, pH 5.6), after which the incisor was rinsed with distilled water (1 minute) and then immersed in cold tea for 3 hr. The incisor was then rinsed with distilled water for 1 min, immersed in pooled filtered human saliva for 1 hr at 37°C, rinsed with distilled water for 1 min, subjected to a 2 min application of chlorhexidine (1%), rinsed with distilled water for 1 min and then immersed in cold tea overnight. The procedure was repeated daily for 7 days. At the end of the 7 days, the colour of the tooth was determined using the chromameter. The degree of colour change was calculated using the formula:

$$E^\star ab = \sqrt{[\Delta L^2 + \Delta a^2 + \Delta b^2]}$$

in which

E* ab is colour difference
L is degree of black/white colour
a is degree of green/red colour
b is degree of blue/yellow colour.

In each treatment group, 5 incisors were used.

The procedure was then repeated, but using a solution containing chlorhexidine (1%) and PVP (av. mol. wt. 40,000, at 5, 10, 15 and 20%) instead of chlorhexidine alone. In addition, a control was run in which the molar was treated with neither chlorhexidine or PVP. The results are presented graphically as Figure 1. The solutions comprising 15 and 20% concentrations of PVP were found to be totally effective in reducing the formation of the CHX/tea stain. There was also a trend towards efectiveness at lower levels of PVP.

In a second set of experiments, solutions containing chlorhexidine digluconate (1%) and various amounts of PVP (M W 30000 - 10, 15 and 20%;and M W 40000 - 20%) in sodium acetate (0.1M) at pH 5.6 were tested as above. The results are presented graphically as Figure 2. The treatment of bovine enamel with PVP (20%, MW = 40,000; 15%, MW = 30,000; 20%, MW = 30,000) was found to result in significantly less stain formation than treatment with chlorhexidine alone. The reduction in stain using PVP (10%, MW = 30,000) was not however significantly significant.

**Example 2** - The impact of polyvinylpyrrolidone on the antibacterial activity of chlorhexidine was evaluated as follows:

Solutions of chlorhexidine (1%) were tested in the presence and absence of PVP (10%, molecular weights of 10,000, 30,000 and 40,000) for microbiological activity using *S. sanguis* as the test organism. Polyvinyl pyrrolidone alone was found to have no antibacterial activity. PVP (10% solution, Mol wt. 30,000 and 40,000) had no significant effect on the activity of the chlorhexidine whilst PVP(10% solution, mol wt 10,000) reduced activity by 12% (significance p=0.019).

When the assessment was extended to higher concentrations of PVP, PVP (15 and 20%, mol wt 30,000 and 40,000) was found to have a significant effect on the activity of chlorhexidine against the test organism *S.sanguis.* PVP (mol wt 30,000) (15% and 20%) reduced activity by 20% and 28% respectively. PVP (mol wt 40,000) (15 and 20%) reduced activity by 38% and 32% respectively. Against the test organism *B. ureolyticus,* PVP (15 and 20%)(mol wt 30,000) significantly reduced activity by 26 and 20% respectively. PVP (mol wt 40000) significantly reduced activity by 14% when tested at 20% but not at 15%.

**Example 3 - toothpaste**

| | |
|---|---|
| Glycerine | 8.0% |
| Hydroxypropyl methylcellulose[a] | 3.40 |
| Polyvinyl pyrrolidone[b] | 20.00 |
| Chlorhexidine digluconate (20%) | 5.30 |
| Sodium saccharin | 0.10 |
| Silica[c] | 16.00 |
| Polyoxyethylene-polyoxypropylene block copolymer[d] | 2.00 |
| Sodium fluoride | 0.22 |
| Talin | 0.02 |
| Flavour | 1.00 |
| Deionised water | qs |

[a] Methocel K15M premium and Methocel K100LV premium in ratio 1:4.

[b] average molecular weight 30,000

[c] RP93 from Rhone-Poulenc

[d] Pluronic F108 (BASF-Wyandolte).

Claims

1. A dentifrice composition comprising a bacteriostatic effective amount of a cationic anti-bacterial agent, an anti-stain effective amount of an anti-staining agent and an orally acceptable carrier or excipient in which the composition is a dentifrice in which the surfactant, thickening agent and abrasive are selected for compatability with the cationic anti-bacterial agent;
characterised in that the anti-staining agent is polyvinyl pyrrolidone which is present in from at least 1 **%** by weight of the composition; and excluding dentifrices in which the cationic antibacterial agent is benzalkonium chloride and dentifrices further comprising an acid of the general formula $RCH_2CONHOH$ in which R is an alkyl group or an alkoxyphenyl group.

2. A dentifrice as claimed in claim 1 which comprises a non-ionic, cationic or amphoteric surfactant, or a mixture thereof; a non-ionic thickening agent and an abrasive which is either an essentially insoluble compound and substantially devoid of water soluble anionic impurities or a sparingly soluble compound used in conjunction with an agent to suppress anion formation, or a mixture thereof.

3. A dentifrice as claimed in claim 1 or 2 in which the surfactant comprises a non-ionic surfactant.

4. A dentifrice as claimed in any one of claims 1 to 3 in which the abrasive is a low-anion silica abrasive.

5. An oral hygiene composition, other than a mouthwash, a dentifrice or denture cleaning tablets, comprising a bacteriostatic effective amount of a cationic anti-bacterial agent, an anti-stain effective amount of an anti-staining agent and an orally acceptable carrier or excipient characterised in that the anti-staining agent is polyvinyl pyrrolidone.

6. A composition as claimed in claim 5 in which polyvinyl pyrrolidone is present in from at least 1 **%** by weight of the composition.

7. A composition as claimed in any one of claims 1 to 6 in which polyvinyl pyrrolidone has an average molecular weight in the range 5,000 to 100,000.

8. A composition as claimed in any one of claims 1 to 7 in which polyvinyl pyrrolidone is present in from 2 to 30% by weight of the composition.

9. A composition as claimed in claim 8 in which polyvinyl pyrrolidone is present in from 5 to 25 **%** by weight of the composition.

10. A composition as claimed in any one of claims 1 to 9 in which the cationic antibacterial agent is a polybiguanide, a quaternary ammonium compound, a prydinium compound, a *bis*-pyridinamine compound, a pyrimidine compound, an amidine compound or an orally acceptable acid addition salt thereof.

11. A composition as claimed in claim 10 in which the cationic antibacterial agent is selected from chlorhexidine, alexidine, octenidine, hexitidine, hexamidine or an orally acceptable acid addition salt thereof, an orally acceptable cetyltrimethylammonium salt, an orally acceptable benzalkonium chloride salt, an orally acceptable benzethonium salt or an orally acceptable cetyl pyridinium salt.

12. An oral hygiene composition as defined in any one of the preceding claims for use in therapy.

13. The use of polyvinyl pyrrolidone and a cationic anti-bacterial agent in the manufacture of an oral hygiene composition for use in oral hygiene with reduced staining.

14. An oral hygiene kit comprising as a first item an oral hygiene composition comprising an anti-stain effective amount of polyvinyl pyrrolidone and an orally acceptable carrier or excipient and, as a separate second item, an oral hygiene composition comprising a bacteriostatic effective amount of a cationic antibacterial agent and an orally acceptable carrier or excipient.

15. An oral hygiene kit as claimed in claim 14 for use in therapy.

16. The use of polyvinyl pyrrolidone in the manufacture of an oral hygiene composition for use, separately, simultaneously or sequentially, with an oral hygiene composition comprising a bacteriostatic effective amount of a cationic anti-bacterial agent, to reduce or eliminate the stain associated with the use of an oral hygiene composition comprising a cationic anti-bacterial agent.

17. A process for preparing an oral hygiene composition as defined in any one of claims 1 to 11 which process comprises admixing the ingredients in the appropriate amounts in any order that is convenient and, if necessary, adjusting the pH to the final required value.